# EUROPEAN PATENT SPECIFICATION

(11) **EP 2 266 596 B1**
(45) Date of publication and mention of the grant of the patent: **02.07.2014**
(21) Application number: 08767349.7
(22) Date of filing: 25.03.2008
(51) Int. Cl.: A61K 38/28, A61K 9/14, A61J 3/02, A61K 47/36

(54) **PHARMACEUTICAL COMPOSITION AND A METHOD FOR THE PRODUCTION THEREOF**
PHARMAZEUTISCHE ZUSAMMENSETZUNG UND VERFAHREN ZU IHRER HERSTELLUNG
COMPOSITION PHARMACEUTIQUE ET PROCÉDÉ DE FABRICATION CORRESPONDANT

(30) Priority: 19.03.2008 UA 2008003496
(43) Date of publication of application: 29.12.2010
(73) Proprietor: SHISHKUV MLYN, a.s., (CZ), 11000 (CZ)
(72) Inventor: Noga, David Anatol'evich, kv. 23 Kiev, 04080 (UA); Matvieev, Pavel Gueorguievich, kv. 185, Kiev, 02068 (UA); Markin, Serguei Sergueevich, kv. 85, Moscow, 125445 (RU); Berenshtein, Dmitrij Borisovich, kv.33, Moscow, 125080 (RU); Siemienov, Mikhail Pietrovich, kv. 33, Moscow, 117133 (RU); Tarasov, Alexandr Andreevich, kv. 9, Kiev, 01025 (UA); Tarasova, Ol'ga Maratovna, kv. 9, Kiev, 01025 (US); Red'Kin, Igor Viacheslavovich, kv. 6, Kiev, 02068 (UA)
(74) Representative: Meissner, Peter E.
(86) International application number: PCT/UA2008/000019
(87) International publication number: WO 2009/116960

(56) References cited:
- EP-A2- 1 891 943
- WO-A1-2008/028264
- RU-C1- 2 066 551
- RU-C1- 2 147 226
- RU-C2- 2 256 440
- RU-C2- 2 291 686
- UA-C2- 24 452
- US-A- 5 554 388
- US-A- 5 554 388

## Description

The invention relates to producing medicinal agents comprising an active operating substance, bound to the carrier, and can be used for obtaining preparations with controlled release of insulin.

Recently the great attention has been given to the development of the pharmaceutical preparations providing for controlled release of operating substance, selecting carriers for active medicinal agents and developing methods for producing preparations in a medicinal form to be suitable for a patient administration.

Insulin is one of the active medicinal substances, which such developments are especially necessary for.

There is known a method for producing a stable dry powdery insulin - containing composition for peroral administration, which comprises the step of dissolving insulin and a pharmaceutical carrier in a water buffer followed by spray-drying the solution to obtain amorphous particles, the average size of which ranges from 0.1 to 10 mkm and the moisture content is less than 10 %, thus as a carrier there are used carbohydrates from the group consisting of mannitol, raffinose, lactose, maltodextrin, or sodium organic salts such as citrate, gluconate, ascorbate (RU 2175556, 20.02.1999).

However the obtained powdery agent is only intended for adsorbing insulin through lungs.

There is known a composition for insulin nasal administration wherein as a carrier it is used aggregated crystalline cellulose with specific granulometric distribution (RU 2299743, 27.05.2007).

There is known a pharmaceutical composition for peroral administration containing insulin on polyethylene oxide carrier of molecular mass ranging from 0.4 kDa to 40 kDa. The carrier is preliminary irradiated with a high-energy irradiation, and then insulin is added thereto to obtain its protein concentration of 1 mg/ml to 10 mg/ml and polyethylene oxide - insulin ratio of (1-500:1) and the mixture is agitated to obtain the transparent or slightly opalescence solution (RU 2316339, 10.02.2008).

However the obtained preparation has no property of controlled release of active substance.

To produce medicinal forms of slowed down release, polymeric bioblastable substances are considered the most perspective carriers.

For example, there is known a bioblastable three-block polymer with average molecular mass of 2000-4990 Dalton possessing properties of a reversible thermal gelatinization, that is, existing in the form of an aqueous solution at low temperatures, and forming gel at physiologically suitable temperatures. The polymer contains bioblastable hydrophobic polyester to be prepared on the basis of various monomers as well as a bioblastable hydrophilic polymeric block of polyethylene glycol wherein it is possible to introduce insulin (RU 2232779, 20.07.2004).

There is also known a method to produce microparticles for slowed down release of substance wherein a composition containing an active agent is dispersed in a polymer organic solution, and an obtained mixture is added into external phase in the form of aqueous solution or oil phase, then the solvent is removed under pressure or in a flow of an inert gas. Thus the composition comprising the active agent can contain chitosan with deacylation degree of 25 to100 % and molecular mass of 10000 to 2000000 Da, which is dissolved in acetic acid. As a result of the method realization, there could be obtained spherical microparticles with diameter of 1 to 500 mkm. (RU 2291686, 20.01. 2007).

There is known a method wherein a chitosan derivative is chosen taking into account the type and the number of its charged groups, the form of operating agent is also chosen so that at certain pH level, there was provided matching the charges of the operating agent and the carrier in the preparation. From the chosen chitosan derivative, there is obtained water sol containing operating agent, then the water sol pH value is regulated to achieve its isoionic points (condition) with possible sedimentation of colloidal particles or nanosized operating agent, and the obtained water sol is subjected to drying (RU 2256440, 10.10.2003).

In spite of the fact that the list of the active agents, which are suitable to be used in above said methods, includes insulin, the conditions, which are necessary to obtain a preparation with the slowed down release of insulin for peroral administration, are not disclosed in the given patents. The methods known from the art only remind about the possibility of obtaining nanosized particles for operating agent on a chitosan derivative carrier.

As a prototype for the claimed invention, there is proposed a method of producing a pharmaceutical composition containing insulin on a polysaccharide carrier. The method comprises mixing initial ingredients such as a suspension of chitosan in a phosphatic buffered solution with pH = 7.3 - 7.4 or with pH = 4 and sodium insulin in a phosphatic buffered solution with pH = 7.3 - 7.4 or with pH = 4, the solutions are admixed at volume ratio 1:1, and introducing hydrochloric acid up to obtaining an insulin and chitosan soluble complex with pH = 3.6 - 4.4. The proposed composition is intended for nasal administration or as a vaccine (US 5554388, 10.09.1996).

Disadvantage of the known method is the absence of stability that results in obtaining a composition with a wide scatter of properties. The method is also not suitable for obtaining the medicinal form for peroral administration that is characterized by controlled release of insulin.

Perorally entered preparations pass through esophagus, stomach and intestine (the organs having various acidity of medium and various enzymes) that complicates developing effective means for peroral administration. At peroral administration, insulin is split into amino acids and small peptides which are quickly decayed in epithelium of small bowel by aminopeptidase that weakens action of a preparation and reduces efficiency of controlled release.

The technical problem of the present invention is to develop a method for producing insulin on a carrier for peroral administration providing for production of a pharmaceutical composition of slowed down release, which is characterized by the stability of the properties.

The set technical problem is solved realizing the described method for producing a pharmaceutical composition containing insulin on a polysaccharide carrier, which involves mixing initial ingredients being as positively charged chitosan sol with pH of 3.5 to 4.5 and negatively charged zinc free insulin, which is taken in the form of a colloidal solution or in the form of nanosized crystalline particles, bringing the pH of the mixed sol to a value of 5.5 to 6.5, producing a gel and dehydrating thus produced gel to obtain solid particles, the size of which ranges from 10 to 100 mkm.

Preferably, the step of producing gel is performed at presence of sodium alginate in the mixed sol in the amount not exceeding 5 % of masses, if calculated per solid substance.

It is provided the step of mixing initial ingredients to be carried out based on calculation of insulin - chitosan mass ratio in the mixed sol, which is equal to (0.2-0.8):1, under condition of the mixed sol electrical neutrality.

Preferably, while mixing, there is supplied a positively charged chitosan sol in a buffered acetate solution with the size of chitosan particles of 200 to 400 nanometers.

Preferably, while mixing, there is supplied a positively charged chitosan sol in the form of its chlorohydrate having molecular mass of 80 to 120 kDa and deacetylation degree of 85 to 89 %.

While mixing, there is supplied insulin in the form of crystalline particles, the sizes of which are ranged from 800 to 1200 nanometers or as a colloidal solution of insulin in the phosphatic buffer with pH of 8.0 to 9.0.

The most preferably, while mixing there is supplied chromatography cleaned gene-engineered insulin obtained with the use of STRAIN ESCHERICHIA COLI XLI-BLUE/PINSR as PREPROINSULIN PRODUCENT.

The set technical problem is also solved with the help of the claimed pharmaceutical composition of slowed down release for the peroral administration, which contains insulin on the carrier-chitosan obtained with the use of the method characterized above.

The parameters of the method proposed for obtaining the composition after the invention are experimentally specified. The essential features of the method to be worded in the independent claim of the invention are necessary for achieving sufficient stability for the carrier-chitosan sol, obtaining the stable mixed nanosized sol of high homogeneity degree and with its ability to have been transformed into gel state up to obtaining a stable and highly structured xerogel transformable into nanosized particles at keeping activity of the operating agent. While applying the obtained xerogel particles, it is possible to produce medicinal forms by means of known methods, both in the form of tablets and pills, and in the form of capsules as well.

Further there exposed the concrete examples of the invention realization.

### Example 1.

To obtain the positively charged chitosan sol, there used the chitosan chlorohydrate particles having molecular mass of 100 kDa, deacetylation degree up to 87 %, the average particle size of 300 nanometers and zeta-potential +50 mV.

The chosen chitosan particles are intensively agitated with acetate buffered solution (pH=4.0) prepared using bidistilled water, providing for the mol ratio of hydrogen ion (H ⁺) : glucosamine chitosan link (GlcN) equal to (0.5-1:1. The number of positively charged centers on chitosan surface (positive charge density) depends on degree of protonation of chitosan amino groups. The sol particle zeta-potential has made +50 mV.

Na-insulin is obtained with the use of STRAIN ESCHERICHIA COLI XLI-BLUE/PINSR as PREPROINSULIN PRODUCENT according to patents RU 2148642, 2000 and UA 24452, 2003.

50 ml of the obtained positively charged chitosan sol of 10 mg/ml concentration are mixed with 50 ml of the colloidal solution of negatively charged Na-insulin suspended in the phosphatic buffered solution with pH=9.0, at insulin concentration of 5 mg/ml, that is at insulin : chitosan mass ratio equal to 0.5: 1. The pH value of the mixed sol is brought up to 6.0 and kept under such a condition for 10 minutes to obtain the stable electrically neutral gel.

The obtained gel is subjected to the process of dehydration by sublimating drying resulted in obtaining xerogel with the particle size of 10 to 50 mkm.

As a result, there is produced a pharmaceutical composition, which is an insulin and chitosan complex with insulin amount of 190 IU/ml. The above said composition possesses an effect of the slowed down release, and it is recommended for peroral administration.

### Example 2.

The method is performed with the use of the positively charged chitosan acetate sol having molecular mass of 80 kDa, deacetylation degree of 85 %, and the average particle size of 400 nanometers, which are dispersed in the acetate buffer, the pH value of which makes 3.5.

The negatively charged crystalline particles of human biosynthetic insulin sized of 800 to 1200 nanometers are introduced into chitosan sol at intensive agitation and at insulin: chitosan mass ratio equal to 0.2:1. After that there is added sodium alginate in amount of 5 % by mass, then there is added alkali in amount, which is sufficient to obtain the pH value of 5.5, and the process is followed with further agitation within 3 minutes. The obtained electrically neutral gel is subjected to pulverization drying. As a result, there are produced solid particles of the insulin and chitosan complex sized of 50 to100 mkm.

### Example 3.

The method is performed as in example 1 with the use of the positively charged chitosan glutamate sol having molecular mass of 120 kDa, deacetylation degree of 89 %, the average particle size of 200 nanometers, which are dispersed in the acetate buffer, the pH value of which makes 4.5. The sol particle zeta-potential has made +35 mV.

The colloid solution of pork insulin in the phosphatic buffer with pH 9.0 is supplied for mixing with the sol at insulin: chitosan mass ratio equal to 0.8:1. After achieving the pH value of 6.5 for the mixed sol and keeping the system under such a condition up to bringing it to the state when it is possible to obtain electrically neutral and stable gel, the product is crushed with the use of a ball-valve mill and dried up. As a result, there is produced an insulin and chitosan complex with insulin amount of 220 IU/ml and particle size of 10 to 100 mkm. The above said complex possesses an effect of slowed down release (of active agent).

The obtained pharmaceutical composition has been checked up on mice.

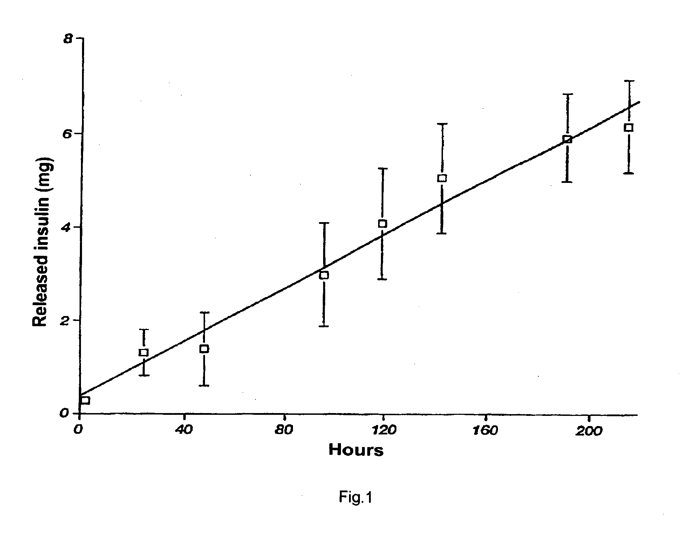

Monitoring kinetics of insulin releasing from the obtained pharmaceutical composition is performed by means of a highly effective liquid and reverse - phase chromatography with gradient elution. Data on continuous release of insulin are presented in fig. 1 in the form of graphic dependence of insulin amount on time.

The results of the analysis of the inventive solutions have exposed the high efficiency of the insulin - chitosan complex for peroral administration produced according to the claimed method. The obtained agent has exposed the effective controlled release of insulin during 7-8 days.

## Claims

1. A method for producing a pharmaceutical composition containing insulin on a polysaccharide carrier, which involves mixing initial ingredients, ***characterized in*** supplying, for mixing, positively charged chitosan sol with pH of 3.5 to 4.5 and negatively charged zinc free insulin, which is taken in the form of a colloidal solution or in the form of nanosized crystalline particles, bringing the pH of the mixed sol to a value of 5.5 to 6.5, producing a gel and dehydrating the produced gel to obtain solid particles, the size of which ranges from 10 to 100 mkm.

2. A method according to claim 1, ***characterized in* that** the gel is produced at presence of sodium alginate in the mixed sol in the amount not exceeding 5% of masses, if calculated per solid substance.

3. A method according to claim 1, ***characterized in* that** the initial ingredients are mixed based on the results of calculation of insulin - chitosan mass ratio in the mixed sol to be equal to (0.2-0.8):1, under condition of the mixed sol electrical neutrality.

4. A method according to claim 1, ***characterized in* that**, for mixing, there is supplied positively charged chitosan sol in a buffered acetate solution with the size of chitosan particles of 200 to 400 nanometers.

5. A method according to claim 1, ***characterized in* that**, for mixing, there is supplied positively charged chitosan sol in the form of its chlorohydrate having molecular mass of 80 to 120 kDa and deacetylation degree of 85 to 89 %.

6. A method according to claim 1, ***characterized in* that**, for mixing, there is supplied insulin in the form of crystalline particles, the sizes of which are ranged from 800 to 1200 nanometers.

7. A method according to claim 1, ***characterized in* that**, for mixing, there is supplied a colloidal solution of insulin in the phosphatic buffer with pH of 8.0 to 9.0.

8. A method according to claim 1, ***characterized in* that**, for mixing, there is supplied chromatography cleaned gene-engineered insulin obtained with the use of STRAIN ESCHERICHIA COLI XLI-BLUE/PINSR as PREPROINSULIN PRODUCENT.

9. A pharmaceutical composition of slowed down release for peroral administration, which contains insulin on a carrier-chitosan, ***characterized in* that** it is obtained with the use of the method **characterized in** claims 1 - 7.

## Patentansprüche

1. Verfahren zur Herstellung einer pharmazeutischen Zusammensetzung, die Insulin auf einem Polysaccharid-Träger enthält, welches das Mischen der Ausgangsstoffe umfasst, **dadurch gekennzeichnet, dass** zum Mischen ein positiv geladenes Chitosansol mit einem pH-Wert von 3,5 bis 4,5 und ein negativ geladenes zinkfreies Insulin, das in Form einer kolloidalen Lösung oder in Form von nanokristallinen Partikeln verwendet wird, zugeführt werden, dass der pH-Wert des gemischten Sols auf 5,5 bis 6,5 eingestellt wird, dass ein Gel erzeugt wird und dass durch Dehydratation des erzeugten Gels Feststoffteilchen erhalten werden, deren Größe in einem Bereich von 10 bis 100 mkm liegt.

2. Verfahren nach Anspruch 1, **dadurch gekennzeichnet, dass** das Gel in Gegenwart von nicht über 5 % Natriumalginat bezogen auf die berechnete Masse des Feststoffs in dem gemischten Sol hergestellt wird.

3. Verfahren nach Anspruch 1, **dadurch gekennzeichnet, dass** die Ausgangsstoffe auf der Basis der Ergebnisse der Berechnung des Massenverhältnisses von Insulin zu Chitosan von (0.2-0.8): 1 in dem gemischten Sol unter der Bedingung der elektrischen Neutralität des Sols gemischt werden.

4. Verfahren nach Anspruch 1, **dadurch gekennzeichnet, dass** zum Mischen positiv geladenes Chitosansol in einer gepufferten Acetat-Lösung zugeführt wird, wobei die Größe der Chitosanpartikel 200 bis 400 Nanometer beträgt.

5. Verfahren nach Anspruch 1, **dadurch gekennzeichnet, dass** zum Mischen positiv geladenes Chitosansol in Form seines Chlorhydrats mit einer Molekülmasse von 80 bis 120 kDa und einem Deacetylierungsgrad von 85 bis 89 % zugeführt wird.

6. Verfahren nach Anspruch 1, **dadurch gekennzeichnet, dass** zum Mischen Insulin in Form von kristallinen Partikeln zugeführt wird, deren Größen im Bereich von 800 bis 1200 Nanometern liegen.

7. Verfahren nach Anspruch 1, **dadurch gekennzeichnet, dass** zum Mischen eine kolloidale Lösung von Insulin in einem Phosphatpuffer mit einem pH-Wert von 8.0 bis 9.0 zugeführt wird.

8. Verfahren nach Anspruch 1, **dadurch gekennzeichnet, dass** zum Mischen mittels Chromatographie gereinigtes, mit Einsatz von STRAIN ESCHERICHIA COLI XLI-BLUE/PINSR als PREPROINSULIN PRODUZENT gentechnisch verändertes Insulin zugeführt wird.

9. Pharmazeutische Zusammensetzung mit verzögerter Freisetzung zur peroralen Verabreichung, die Insulin auf einem Chitosanträger enthält, **dadurch gekennzeichnet, dass** sie unter Einsatz des in den Ansprüchen 1-7 gekennzeichneten Verfahrens erhalten wird.

## Revendications

1. Procédé de production d'une composition pharmaceutique contenant de l'insuline sur un support polysaccharidique, procédé dans lequel on mélange les ingrédients de départ, ***caractérisé* en ce qu'**il consiste, pour le mélange, à apporter un sol de chitosane chargé positivement ayant un pH de 3,5 à 4,5 et de l'insuline exempte de zinc chargée négativement, qui est prélevée sous la forme d'une solution colloïdale ou sous la forme de particules cristallines de dimension nanométrique, à porter la valeur du pH du sol mélangé entre 5,5 et 6,5, à produire un gel et à déshydrater le gel produit pour obtenir des particules solides dont la dimension est comprise entre 10 et 100 mkm.

2. Procédé selon la revendication 1, ***caractérisé en* ce que** le gel est produit en présence d'alginate de sodium dans le sol mélangé dans une quantité n'excédant pas 5 % des masses, si le calcul est effectué par substance solide.

3. Procédé selon la revendication 1, ***caractérisé en* ce que** les ingrédients de départ sont mélangés sur la base des résultats du calcul du rapport en masse de l'insuline au chitosane dans le sol mélangé comme étant égal à (0,2 à 0,8):1, à condition que le sol mélangé soit électriquement neutre.

4. Procédé selon la revendication 1, ***caractérisé en* ce que**, pour le mélange, du sol de chitosane chargé positivement est fourni dans une solution d'acétate tamponnée, la taille des particules de chitosane étant comprise entre 200 et 400 nanomètres.

5. Procédé selon la revendication 1, ***caractérisé en* ce que**, pour le mélange, du sol de chitosane chargé positivement est fourni sous la forme de son chlorhydrate ayant une masse moléculaire de 80 à 120 kDa et un degré de désacétylation de 85 à 89%.

6. Procédé selon la revendication 1, ***caractérisé en* ce que**, pour le mélange, de l'insuline est fournie sous la forme de particules cristallines dont la taille est comprise entre 800 et 1 200 nanomètres.

7. Procédé selon la revendication 1, ***caractérisé en* ce que**, pour le mélange, une solution colloïdale d'insuline est fournie dans le tampon phosphate ayant un pH de 8,0 à 9,0.

8. Procédé selon la revendication 1, ***caractérisé en* ce que**, pour le mélange, on utilise de l'insuline génétiquement modifiée et nettoyée par chromatographie, obtenue en utilisant la SOUCHE D'ESCHERICHIA COLI XLI-BLUE/PINSR sous forme de PREPROINSULIN PRODUCENT.

9. Composition pharmaceutique à libération retardée pour l'administration par voie perorale, qui contient de l'insuline sur un support chitosane, ***caractérisé en* ce qu'**elle est obtenue en utilisant le procédé selon les revendications 1 à 7.
